# EUROPEAN PATENT APPLICATION

(11) **EP 2 065 476 A1**
(43) Date of publication of application: **03.06.2009**
(21) Application number: 07291429.4
(22) Date of filing: 30.11.2007
(51) Int. Cl.: C12Q 1/68

(54) **A mammalian cell-based screening assay to identify inhibitors of alphaviruses**

(71) Applicant: INSTITUT PASTEUR, 75015 Paris (FR); Centre National de la Recherche Scientifique, 75016 Paris (FR)
(72) Inventor: Vidalain, Pierre-Olivier, 92800 Puteaux (FR); Tangy, Frédéric, 93260 Les Lilas (FR); Jacob, Yves, 28130 Maintenon (FR); Lucas-Hourani, Marianne, 75017 Paris (FR)
(74) Representative: Thomas, Dean

(57) **Abstract**

The current invention concerns a method to identify modulators of virulence of an *Alphavirus,* comprising the steps of i) expressing an *Alphavirus* non-structural protein 2 (nsP2) coding sequence in at least one subject mammalian cell *in vitro*; (ii-a) introducing a first reporter gene under the transcriptional control of an inducible promoter into said at least one subject mammalian cell *in vitro*; (iii) monitoring the activity of said first reporter gene *in vitro*; (iv) comparing said activity of said first reporter gene of step (iii) to at least one control mammalian cell not expressing said nsP2 coding sequence and comprising said first reporter gene under the transcriptional control of said inducible promoter *in vitro.*

SEQUENCE LISTING

<110> Institut Pasteur
Centre National de la Recherche Scientifique

<120> A mammalian cell-based screening assay to identify inhibitors of
Alphavirus

<130> F226-147-EP

<160> 14

<170> PatentIn version 3.3

<210> 1
<211> 2394
<212> DNA
<213> Chikungunya virus

<400> 1
ggaataatag agactccgag aggagctatc aaagttactg cccaaccaac agaccacgtc 60

gtgggagagt acctggtact ctccccgcag accgtactac gtagccagaa gctcagtctg 120

attcacgctt tggcggagca agtgaagacg tgcacgcaca acggacgagc agggaggtat 180

gcggtcgaag cgtacgacgg ccgagtccta gtgccctcag gctatgcaat ctcgcctgaa 240

gacttccaga gtctaagcga aagcgcaacg atggtgtata acgaaagaga gttcgtaaac 300

agaaagctac accatattgc gatgcacgga ccagccctga acaccgacga agagtcgtat 360

gagctggtga gggcagagag gacagaacac gagtacgtct acgacgtgga tcagagaaga 420

tgctgtaaga aggaagaagc cgcaggactg gtactggtgg gcgacttgac taatccgccc 480

taccacgaat tcgcatatga agggctaaaa atccgccctg cctgcccata caaaattgca 540

gtcataggag tcttcggagt accgggatct ggcaagtcag ctattatcaa gaacctagtt 600

accaggcagg acctggtgac tagcggaaag aaagaaaact gccaagaaat caccaccgac 660

gtgatgagac agagaggtct agagatatct gcacgtacgg ttgactcgct gctcttgaat 720

ggatgcaaca gaccagtcga cgtgttgtac gtagacgagg cgtttgcgtg ccactctgga 780

acgctacttg ctttgatcgc cttggtgaga ccaaggcaga aagttgtact ttgtggtgac 840

ccgaagcagt gcggcttctt caatatgatg cagatgaaag tcaactataa tcacaacatc 900

tgcacccaag tgtaccacaa aagtatctcc aggcggtgta cactgcctgt gaccgccatt 960

gtgtcatcgt tgcattacga aggcaaaatg cgcactacga atgagtacaa caagccgatt 1020

gtagtggaca ctacaggctc aacaaaacct gaccctggag acctcgtgtt aacgtgcttc 1080

agagggtggg ttaaacaact gcaaattgac tatcgtggat acgaggtcat gacagcagcc 1140

gcatcccaag ggttaaccag aaaaggagtt tacgcagtta gacaaaaagt taatgaaaac 1200

ccgctctatg catcaacgtc agagcacgtc aacgtactcc taacgcgtac ggaaggtaaa 1260

ctggtatgga agacactttc cggcgacccg tggataaaga cgctgcagaa cccaccgaaa 1320

ggaaacttca aagcaactat taaggagtgg gaggtggagc atgcatcaat aatggcgggc 1380

atctgcagtc accaaatgac cttcgataca ttccaaaata aagccaacgt ttgttgggct 1440

aagagcttgg tccctatcct cgaaacagcg gggataaaac taaatgatag gcagtggtct 1500
cagataattc aagccttcaa agaagacaaa gcatactcac ctgaagtagc cctgaatgaa 1560
atatgtacgc gcatgtatgg ggtggatcta gacagcgggc tattttctaa accgttggtg 1620
tctgtgtatt acgcggataa ccactgggat aataggcctg gagggaaaat gttcggattt 1680
aaccccgagg cagcatccat tctagaaaga aagtatccat tcacaaaagg gaagtggaac 1740
atcaacaagc agatctgcgt gactaccagg aggatagaag actttaaccc taccaccaac 1800
atcataccgg ccaacaggag actaccacac tcattagtgg ccgaacaccg cccagtaaaa 1860
ggggaaagaa tggaatggct ggttaacaag ataaacggcc accacgtgct cctggtcagt 1920
ggctataacc ttgcactgcc tactaagaga gtcacttggg tagcgccgtt aggtgtccgc 1980
ggagcggact acacatacaa cctagagttg ggtctgccag caacgcttgg taggtatgac 2040
ctagtggtca taaacatcca cacacctttt cgcatacacc attaccaaca gtgcgtcgac 2100
cacgcaatga aactgcaaat gctcgggggt gactcattga gactgctcaa accgggcggc 2160
tctctattga tcagagcata tggttacgca gatagaacca gtgaacgagt catctgcgta 2220
ttgggacgca agtttagatc gtctagagcg ttgaaaccac catgtgtcac cagcaacact 2280
gagatgtttt tcctattcag caactttgac aatggcagaa ggaatttcac aactcatgtc 2340
atgaacaatc aactgaatgc agccttcgta ggacaggtca cccgagcagg atgt 2394

<210> 2
<211> 2393
<212> DNA
<213> Chikungunya virus

<400> 2
ggaataatag agactccgag aggagttatc aaagttactg cccaaccaac agaccacgtc 60
gtgggagagt acctggtact ctccccgcag accgtactac gtagccagaa gctcagtctg 120
attcacgctt tggcggagca agtgaagacg tgcacgcaca acggacgagc agggaggtat 180
gcggtcgaag cgtacgacgg ccgagtccta gtgccctcag gctatgcaat ctcgcctgaa 240
gacttccaga gtctaagcga aagcgcaacg atggtgtata acgaaagaga gttcgtaaac 300
agaaagctac accatattgc gatgcacgga ccagccctga acaccgacga agagtcgtat 360
gagctggtga gggcagagag gacagaacac gagtacgtct acgacgtgga tcagagaaga 420
tgctgtaaga aggaagaagc cgcaggactg gtactggtgg gcgacttgac taatccgccc 480
taccacgaat tcgcatatga agggctaaaa atccgccctg cctgcccata caaaattgca 540
gtcataggag tcttcggagt accgggatct ggcaagtcag ctattatcaa gaacctagtt 600
accaggcagg acctggtgac tagcggaaag aaagaaaact gccaagaaat caccaccgac 660
gtgatgagac agagaggtct agagatatct gcacgtacgg ttgactcgct gctcttgaat 720
ggatgcaaca gaccagtcga cgtgttgtac gtagacgagg cgtttgcgtg ccactctgga 780
acgctacttg ctttgatcgc cttggtgaga ccaaggcaga aagttgtact ttgtggtgac 840
ccgaagcagt gcggcttctt caatatgatg cagatgaaag tcaactataa tcacaacatc 900

tgcacccaag tgtaccacaa aagtatctcc aggcggtgta cactgcctgt gaccgccatt 960
gtgtcatcgt tgcattacga aggcaaaatg cgcactacga atgagtacaa caagccgatt 1020
gtagtggaca ctacaggctc aacaaaacct gaccctggag acctcgtgtt aacgtgcttc 1080
agagggtggg ttaaacaact gcaaattgac tatcgtggat acgaggtcat gacagcagcc 1140
gcatcccaag ggttaaccag aaaaggagtt tacgcagtta gacaaaaagt taatgaaaac 1200
ccgctctatg catcaacgtc agagcacgtc aacgtactcc taacgcgtac ggaaggtaaa 1260
ctggtatgga agacactttc cggcgacccg tggataaaga cgctgcagaa cccaccgaaa 1320
ggaaacttca aagcaactat taaggagtgg gaggtggagc atgcatcaat aatggcgggc 1380
atctgcagtc accaaatgac cttcgataca ttccaaaata aagccaacgt ttgttgggct 1440
aagagcttgg tccctatcct cgaaacagcg gggataaaac taaatgatag gcagtggtct 1500
cagataattc aagccttcaa agaagacaaa gcatactcac ctgaagtagc cctgaatgaa 1560
atatgtacgc gcatgtatgg ggtggatcta gacagcgggc tattttctaa accgttggtg 1620
tctgtgtatt acgcggataa ccactgggat aataggcctg gagggaaaat gttcggattt 1680
aaccccgagg cagcatccat tctagaaaga aagtatccat tcacaaaagg gaagtggaac 1740
atcaacaagc agatctgcgt gactaccagg aggatagaag actttaaccc taccaccaac 1800
atcataccgg ccaacaggag actaccacac tcattagtgg ccgaacaccg cccagtaaaa 1860
ggggaaagaa tggaatggct ggttaacaag ataaacggcc accacgtgct cctggtcagt 1920
ggctataacc ttgcactgcc tactaagaga gtcacttggg tagcgccgtt aggtgtccgc 1980
ggagcggact acacatacaa cctagagttg ggtctgccag caacgcttgg taggtatgac 2040
ctagtggtca taaacatcca cacacctttt cgcatacacc attaccaaca gtgcgtcgac 2100
cacgcaatga aactgcaaat gctcgggggt gactcattga gactgctcaa accgggcggc 2160
tctctattga tcagagcata tggttacgca gatagaacca gtgaacgagt catctgcgta 2220
ttgggacgca agtttagatc gtctagagcg ttgaaaccac catgtgtcac cagcaacact 2280
gagatgtttt tcctattcag caactttgac aatggcagaa ggaatttcac aactcatgtc 2340
atgaacaatc aactgaatgc agccttcgta ggacaggcca cccgagcagg atg 2393

<210> 3
<211> 799
<212> PRT
<213> Semliki Forest virus

<400> 3

Gly Val Val Glu Thr Pro Arg Ser Ala Leu Lys Val Thr Ala Gln Pro
1               5                   10                  15

Asn Asp Val Leu Leu Gly Asn Tyr Val Val Leu Ser Pro Gln Thr Val
            20                  25                  30

Leu Lys Ser Ser Lys Leu Ala Pro Val His Pro Leu Ala Glu Gln Val

35                  40                  45

Lys Ile Ile Thr His Asn Gly Arg Ala Gly Arg Tyr Gln Val Asp Gly
    50                  55                  60

Tyr Asp Gly Arg Val Leu Leu Pro Cys Gly Ser Ala Ile Pro Val Pro
65                  70                  75                  80

Glu Phe Gln Ala Leu Ser Glu Ser Ala Thr Met Val Tyr Asn Glu Arg
                85                  90                  95

Glu Phe Val Asn Arg Lys Leu Tyr His Ile Ala Val His Gly Pro Ser
            100                 105                 110

Leu Asn Thr Asp Glu Glu Asn Tyr Glu Lys Val Arg Ala Glu Arg Thr
        115                 120                 125

Asp Ala Glu Tyr Val Phe Asp Val Asp Lys Lys Cys Cys Val Lys Arg
    130                 135                 140

Glu Glu Ala Ser Gly Leu Val Leu Val Gly Glu Leu Thr Asn Pro Pro
145                 150                 155                 160

Phe His Glu Phe Ala Tyr Glu Gly Leu Lys Ile Arg Pro Ser Ala Pro
                165                 170                 175

Tyr Lys Thr Thr Val Val Gly Val Phe Gly Val Pro Gly Ser Gly Lys
            180                 185                 190

Ser Ala Ile Ile Lys Ser Leu Val Thr Lys His Asp Leu Val Thr Ser
        195                 200                 205

Gly Lys Lys Glu Asn Cys Gln Glu Ile Val Asn Asp Val Lys Lys His
    210                 215                 220

Arg Gly Leu Asp Ile Gln Ala Lys Thr Val Asp Ser Ile Leu Leu Asn
225                 230                 235                 240

Gly Cys Arg Arg Ala Val Asp Ile Leu Tyr Val Asp Glu Ala Phe Ala
                245                 250                 255

Cys His Pro Gly Thr Leu Leu Ala Leu Ile Ala Leu Val Lys Pro Arg
            260                 265                 270

Ser Lys Val Val Leu Cys Gly Asp Pro Lys Gln Cys Gly Phe Phe Asn
        275                 280                 285

Met Met Gln Leu Lys Val Asn Phe Asn His Asn Ile Cys Thr Glu Val
    290                 295                 300

Cys His Lys Ser Ile Ser Arg Arg Cys Thr Arg Pro Val Thr Ala Ile

305                 310                 315                 320

Val Ser Thr Leu His Tyr Gly Gly Lys Met Arg Thr Thr Asn Pro Cys
                325                 330                 335

Asn Lys Pro Ile Ile Ile Asp Thr Thr Gly Gln Thr Lys Pro Lys Pro
            340                 345                 350

Gly Asp Ile Val Leu Thr Cys Phe Arg Gly Trp Val Lys Gln Leu Gln
        355                 360                 365

Leu Asp Tyr Arg Gly His Glu Val Met Thr Ala Ala Ala Ser Gln Gly
    370                 375                 380

Leu Thr Arg Lys Gly Val Tyr Ala Val Arg Gln Lys Val Asn Glu Asn
385                 390                 395                 400

Pro Leu Tyr Ala Pro Ala Ser Glu His Val Asn Val Leu Leu Thr Arg
                405                 410                 415

Thr Glu Asp Arg Leu Val Trp Lys Thr Leu Ala Gly Asp Pro Trp Ile
            420                 425                 430

Lys Val Leu Ser Asn Ile Pro Gln Gly Asn Phe Thr Ala Thr Leu Glu
        435                 440                 445

Glu Trp Gln Glu Glu His Asp Lys Ile Met Lys Val Ile Glu Gly Pro
    450                 455                 460

Ala Ala Pro Val Asp Ala Phe Gln Asn Lys Ala Asn Val Cys Trp Ala
465                 470                 475                 480

Lys Ser Leu Val Pro Val Leu Asp Thr Ala Gly Ile Arg Leu Thr Ala
                485                 490                 495

Glu Glu Trp Ser Thr Ile Ile Thr Ala Phe Lys Glu Asp Arg Ala Tyr
            500                 505                 510

Ser Pro Val Val Ala Leu Asn Glu Ile Cys Thr Lys Tyr Tyr Gly Val
        515                 520                 525

Asp Leu Asp Ser Gly Leu Phe Ser Ala Pro Lys Val Ser Leu Tyr Tyr
    530                 535                 540

Glu Asn Asn His Trp Asp Asn Arg Pro Gly Gly Arg Met Tyr Gly Phe
545                 550                 555                 560

Asn Ala Ala Thr Ala Ala Arg Leu Glu Ala Arg His Thr Phe Leu Lys
                565                 570                 575

Gly Gln Trp His Thr Gly Lys Gln Ala Val Ile Ala Glu Arg Lys Ile

580                 585                 590

Gln Pro Leu Ser Val Leu Asp Asn Val Ile Pro Ile Asn Arg Arg Leu
        595                 600                 605

Pro His Ala Leu Val Ala Glu Tyr Lys Thr Val Lys Gly Ser Arg Val
    610                 615                 620

Glu Trp Leu Val Asn Lys Val Arg Gly Tyr His Val Leu Leu Val Ser
625                 630                 635                 640

Glu Tyr Asn Leu Ala Leu Pro Arg Arg Asp Val Thr Trp Leu Ser Pro
                645                 650                 655

Leu Asn Val Thr Gly Ala Asp Arg Cys Tyr Asp Leu Ser Leu Gly Leu
            660                 665                 670

Pro Ala Asp Ala Gly Arg Phe Asp Leu Val Phe Val Asn Ile His Thr
        675                 680                 685

Glu Phe Arg Ile His His Tyr Gln Gln Cys Val Asp His Ala Met Lys
    690                 695                 700

Leu Gln Met Leu Gly Gly Asp Ala Leu Arg Leu Leu Lys Pro Gly Gly
705                 710                 715                 720

Ser Leu Leu Met Arg Ala Tyr Gly Tyr Ala Asp Lys Ile Ser Glu Ala
                725                 730                 735

Val Val Ser Ser Leu Ser Arg Lys Phe Ser Ser Ala Arg Val Leu Arg
            740                 745                 750

Pro Asp Cys Val Thr Ser Asn Thr Glu Val Phe Leu Leu Phe Ser Asn
        755                 760                 765

Phe Asp Asn Gly Lys Arg Pro Ser Thr Leu His Gln Met Asn Thr Lys
    770                 775                 780

Leu Ser Ala Val Tyr Ala Gly Glu Ala Met His Thr Ala Gly Cys
785                 790                 795

<210>  4
<211>  807
<212>  PRT
<213>  Sindbis virus

<400>  4

Ala Leu Val Glu Thr Pro Arg Gly His Val Arg Ile Ile Pro Gln Ala
1               5                   10                  15

Asn Asp Arg Met Ile Gly Gln Tyr Ile Val Val Ser Pro Asn Ser Val
            20                  25                  30

Leu Lys Asn Ala Lys Leu Ala Pro Ala His Pro Leu Ala Asp Gln Val
        35                  40                  45

Lys Ile Ile Thr His Ser Gly Arg Ser Gly Arg Tyr Ala Val Glu Pro
    50                  55                  60

Tyr Asp Ala Lys Val Leu Met Pro Ala Gly Gly Ala Val Pro Trp Pro
65                  70                  75                  80

Glu Phe Leu Ala Leu Ser Glu Ser Ala Thr Leu Val Tyr Asn Glu Arg
                85                  90                  95

Glu Phe Val Asn Arg Lys Leu Tyr His Ile Ala Met His Gly Pro Ala
            100                 105                 110

Lys Asn Thr Glu Glu Glu Gln Tyr Lys Val Thr Lys Ala Glu Leu Ala
        115                 120                 125

Glu Thr Glu Tyr Val Phe Asp Val Asp Lys Lys Arg Cys Val Lys Lys
    130                 135                 140

Glu Glu Ala Ser Gly Leu Val Leu Ser Gly Glu Leu Thr Asn Pro Pro
145                 150                 155                 160

Tyr His Glu Leu Ala Leu Glu Gly Leu Lys Thr Arg Pro Ala Val Pro
                165                 170                 175

Tyr Lys Val Glu Thr Ile Gly Val Ile Gly Thr Pro Gly Ser Gly Lys
            180                 185                 190

Ser Ala Ile Ile Lys Ser Thr Val Thr Ala Arg Asp Leu Val Thr Ser
        195                 200                 205

Gly Lys Lys Glu Asn Cys Arg Glu Ile Glu Ala Asp Val Leu Arg Leu
    210                 215                 220

Arg Gly Met Gln Ile Thr Ser Lys Thr Val Asp Ser Val Met Leu Asn
225                 230                 235                 240

Gly Cys His Lys Ala Val Glu Val Leu Tyr Val Asp Glu Ala Phe Ala
                245                 250                 255

Cys His Ala Gly Ala Leu Leu Ala Leu Ile Ala Ile Val Arg Pro Arg
            260                 265                 270

Lys Lys Val Val Leu Cys Gly Asp Pro Met Gln Cys Gly Phe Phe Asn
        275                 280                 285

Met Met Gln Leu Lys Val His Phe Asn His Pro Glu Lys Asp Ile Cys
    290                 295                 300

Thr Lys Thr Phe Tyr Lys Tyr Ile Ser Arg Arg Cys Thr Gln Pro Val
305                 310                 315                 320

Thr Ala Ile Val Ser Thr Leu His Tyr Asp Gly Lys Met Lys Thr Thr
                325                 330                 335

Asn Pro Cys Lys Lys Asn Ile Glu Ile Asp Ile Thr Gly Ala Thr Lys
            340                 345                 350

Pro Lys Pro Gly Asp Ile Ile Leu Thr Cys Phe Arg Gly Trp Val Lys
        355                 360                 365

Gln Leu Gln Ile Asp Tyr Pro Gly His Glu Val Met Thr Ala Ala Ala
    370                 375                 380

Ser Gln Gly Leu Thr Arg Lys Gly Val Tyr Ala Val Arg Gln Lys Val
385                 390                 395                 400

Asn Glu Asn Pro Leu Tyr Ala Ile Thr Ser Glu His Val Asn Val Leu
                405                 410                 415

Leu Thr Arg Thr Glu Asp Arg Leu Val Trp Lys Thr Leu Gln Gly Asp
            420                 425                 430

Pro Trp Ile Lys Gln Pro Thr Asn Ile Pro Lys Gly Asn Phe Gln Ala
        435                 440                 445

Thr Ile Glu Asp Trp Glu Ala Glu His Lys Gly Ile Ile Ala Ala Ile
    450                 455                 460

Asn Ser Pro Thr Pro Arg Ala Asn Pro Phe Ser Cys Lys Thr Asn Val
465                 470                 475                 480

Cys Trp Ala Lys Ala Leu Glu Pro Ile Leu Ala Thr Ala Gly Ile Val
                485                 490                 495

Leu Thr Gly Cys Gln Trp Ser Glu Leu Phe Pro Gln Phe Ala Asp Asp
            500                 505                 510

Lys Pro His Ser Ala Ile Tyr Ala Leu Asp Val Ile Cys Ile Lys Phe
        515                 520                 525

Phe Gly Met Asp Leu Thr Ser Gly Leu Phe Ser Lys Gln Ser Ile Pro
    530                 535                 540

Leu Thr Tyr His Pro Ala Asp Ser Ala Arg Pro Val Ala His Trp Asp
545                 550                 555                 560

Asn Ser Pro Gly Thr Arg Lys Tyr Gly Tyr Asp His Ala Ile Ala Ala
                565                 570                 575

Glu Leu Ser Arg Arg Phe Pro Val Phe Gln Leu Ala Gly Lys Gly Thr
            580                 585                 590

Gln Leu Asp Leu Gln Thr Gly Arg Thr Arg Val Ile Ser Ala Gln His
        595                 600                 605

Asn Leu Val Pro Val Asn Arg Asn Leu Pro His Ala Leu Val Pro Glu
    610                 615                 620

Tyr Lys Glu Lys Gln Pro Gly Pro Val Lys Lys Phe Leu Asn Gln Phe
625                 630                 635                 640

Lys His His Ser Val Leu Val Val Ser Glu Glu Lys Ile Glu Ala Pro
                645                 650                 655

Arg Lys Arg Ile Glu Trp Ile Ala Pro Ile Gly Ile Ala Gly Ala Asp
            660                 665                 670

Lys Asn Tyr Asn Leu Ala Phe Gly Phe Pro Pro Gln Ala Arg Tyr Asp
        675                 680                 685

Leu Val Phe Ile Asn Ile Gly Thr Lys Tyr Arg Asn His His Phe Gln
    690                 695                 700

Gln Cys Glu Asp His Ala Ala Thr Leu Lys Thr Leu Ser Arg Ser Ala
705                 710                 715                 720

Leu Asn Cys Leu Asn Pro Gly Gly Thr Leu Val Val Lys Ser Tyr Gly
                725                 730                 735

Tyr Ala Asp Arg Asn Ser Glu Asp Val Val Thr Ala Leu Ala Arg Lys
            740                 745                 750

Phe Val Arg Val Ser Ala Ala Arg Pro Asp Cys Val Ser Ser Asn Thr
        755                 760                 765

Glu Met Tyr Leu Ile Phe Arg Gln Leu Asp Asn Ser Arg Thr Arg Gln
    770                 775                 780

Phe Thr Pro His His Leu Asn Cys Val Ile Ser Ser Val Tyr Glu Gly
785                 790                 795                 800

Thr Arg Asp Gly Val Gly Ala
                805

<210>  5
<211>  56
<212>  DNA
<213>  Artificial

<220>

<223>  attB1-nsP2-For PCR Primer

<400>  5
ggggacaact ttgtacaaaa aagttggcat gggaataata gagactccga gaggag          56

<210>  6
<211>  48
<212>  DNA
<213>  Artificial

<220>
<223>  attB2-nsP2-Rev PCR Primer

<400>  6
ggggacaact ttgtacaaga aagttggtta acatcctgct cgggtggc                   48

<210>  7
<211>  331
<212>  PRT
<213>  Homo sapiens

<400>  7
   Met Thr Ala Lys Met Glu Thr Thr Phe Tyr Asp Asp Ala Leu Asn Ala
   1               5                   10                  15
   Ser Phe Leu Pro Ser Glu Ser Gly Pro Tyr Gly Tyr Ser Asn Pro Lys
               20                  25                  30
   Ile Leu Lys Gln Ser Met Thr Leu Asn Leu Ala Asp Pro Val Gly Ser
           35                  40                  45
   Leu Lys Pro His Leu Arg Ala Lys Asn Ser Asp Leu Leu Thr Ser Pro
       50                  55                  60
   Asp Val Gly Leu Leu Lys Leu Ala Ser Pro Glu Leu Glu Arg Leu Ile
   65                  70                  75                  80
   Ile Gln Ser Ser Asn Gly His Ile Thr Thr Thr Pro Thr Pro Thr Gln
                   85                  90                  95
   Phe Leu Cys Pro Lys Asn Val Thr Asp Glu Gln Glu Gly Phe Ala Glu
               100                 105                 110
   Gly Phe Val Arg Ala Leu Ala Glu Leu His Ser Gln Asn Thr Leu Pro
           115                 120                 125
   Ser Val Thr Ser Ala Ala Gln Pro Val Asn Gly Ala Gly Met Val Ala
       130                 135                 140
   Pro Ala Val Ala Ser Val Ala Gly Gly Ser Gly Ser Gly Gly Phe Ser
   145                 150                 155                 160
   Ala Ser Leu His Ser Glu Pro Pro Val Tyr Ala Asn Leu Ser Asn Phe
                   165                 170                 175
   Asn Pro Gly Ala Leu Ser Ser Gly Gly Gly Ala Pro Ser Tyr Gly Ala
               180                 185                 190
   Ala Gly Leu Ala Phe Pro Ala Gln Pro Gln Gln Gln Gln Gln Pro Pro
           195                 200                 205
   His His Leu Pro Gln Gln Met Pro Val Gln His Pro Arg Leu Gln Ala
       210                 215                 220
   Leu Lys Glu Glu Pro Gln Thr Val Pro Glu Met Pro Gly Glu Thr Pro
   225                 230                 235                 240
   Pro Leu Ser Pro Ile Asp Met Glu Ser Gln Glu Arg Ile Lys Ala Glu
                   245                 250                 255
   Arg Lys Arg Met Arg Asn Arg Ile Ala Ala Ser Lys Cys Arg Lys Arg
               260                 265                 270
   Lys Leu Glu Arg Ile Ala Arg Leu Glu Glu Lys Val Lys Thr Leu Lys
           275                 280                 285
   Ala Gln Asn Ser Glu Leu Ala Ser Thr Ala Asn Met Leu Arg Glu Gln
       290                 295                 300
   Val Ala Gln Leu Lys Gln Lys Val Met Asn His Val Asn Ser Gly Cys
   305                 310                 315                 320
   Gln Leu Met Leu Thr Gln Gln Leu Gln Thr Phe
                   325                 330

<210>  8
<211>  380
<212>  PRT
<213>  Homo sapiens

<400>  8

Met Met Phe Ser Gly Phe Asn Ala Asp Tyr Glu Ala Ser Ser Ser Arg
1               5                   10                  15

Cys Ser Ser Ala Ser Pro Ala Gly Asp Ser Leu Ser Tyr Tyr His Ser
            20                  25                  30

Pro Ala Asp Ser Phe Ser Ser Met Gly Ser Pro Val Asn Ala Gln Asp
        35                  40                  45

Phe Cys Thr Asp Leu Ala Val Ser Ser Ala Asn Phe Ile Pro Thr Val
    50                  55                  60

Thr Ala Ile Ser Thr Ser Pro Asp Leu Gln Trp Leu Val Gln Pro Ala
65                  70                  75                  80

Leu Val Ser Ser Val Ala Pro Ser Gln Thr Arg Ala Pro His Pro Phe
                85                  90                  95

Gly Val Pro Ala Pro Ser Ala Gly Ala Tyr Ser Arg Ala Gly Val Val
            100                 105                 110

Lys Thr Met Thr Gly Gly Arg Ala Gln Ser Ile Gly Arg Arg Gly Lys
        115                 120                 125

Val Glu Gln Leu Ser Pro Glu Glu Glu Glu Lys Arg Arg Ile Arg Arg
    130                 135                 140

Glu Arg Asn Lys Met Ala Ala Ala Lys Cys Arg Asn Arg Arg Arg Glu
145                 150                 155                 160

Leu Thr Asp Thr Leu Gln Ala Glu Thr Asp Gln Leu Glu Asp Glu Lys
                165                 170                 175

Ser Ala Leu Gln Thr Glu Ile Ala Asn Leu Leu Lys Glu Lys Glu Lys
            180                 185                 190

Leu Glu Phe Ile Leu Ala Ala His Arg Pro Ala Cys Lys Ile Pro Asp
        195                 200                 205

Asp Leu Gly Phe Pro Glu Glu Met Ser Val Ala Ser Leu Asp Leu Thr
    210                 215                 220

Gly Gly Leu Pro Glu Val Ala Thr Pro Glu Ser Glu Glu Ala Phe Thr
225                 230                 235                 240

Leu Pro Leu Leu Asn Asp Pro Glu Pro Lys Pro Ser Val Glu Pro Val
                245                 250                 255

Lys Ser Ile Ser Ser Met Glu Leu Lys Thr Glu Pro Phe Asp Asp Phe
            260                 265                 270

Leu Phe Pro Ala Ser Ser Arg Pro Ser Gly Ser Glu Thr Ala Arg Ser
        275                 280                 285

Val Pro Asp Met Asp Leu Ser Gly Ser Phe Tyr Ala Ala Asp Trp Glu
    290                 295                 300

Pro Leu His Ser Gly Ser Leu Gly Met Gly Pro Met Ala Thr Glu Leu
305                 310                 315                 320

Glu Pro Leu Cys Thr Pro Val Val Thr Cys Thr Pro Ser Cys Thr Ala
                325                 330                 335

Tyr Thr Ser Ser Phe Val Phe Thr Tyr Pro Glu Ala Asp Ser Phe Pro
            340                 345                 350

Ser Cys Ala Ala Ala His Arg Lys Gly Ser Ser Ser Asn Glu Pro Ser
        355                 360                 365

Ser Asp Ser Leu Ser Ser Pro Thr Leu Leu Ala Leu
    370                 375                 380

<210>  9
<211>  425
<212>  PRT
<213>  Homo sapiens

<400>  9

<210>  10
<211>  146
<212>  DNA
<213>  Artificial

<220>
<223>  pGAL4-UAS-Luc minimal promoter

<400>  10
ggggatccgc tcggaggaca gtactccgct cggaggacag tactccgctc ggaggacagt      60

actccgctcg gaggacagta ctccgctcgg aggacagtac tccgatccgt cgactctaga     120

gggtatataa tgcgccagct cgaatt                                          146

<210>  11
<211>  49
<212>  DNA
<213>  Artificial

<220>
<223>  attB1-nsP2sindbis-For primer

<400>  11
ggggacaact ttgtacaaaa aagttggcat ggcattagtt gaaaccccg                 49

<210>  12
<211>  48
<212>  DNA
<213>  Artificial

<220>
<223>  attB2-nsP2sindbis-Rev primer

<400>  12
ggggacaact ttgtacaaga aagttggtta ggctccaact ccatctct                  48

<210>  13
<211>  49
<212>  DNA
<213>  Artificial

<220>
<223>  attB1-nsP2SFV-For primer

<400>  13
ggggacaact ttgtacaaaa aagttggcat gggggtcgtg gaaacacct                 49

<210>  14
<211>  48
<212>  DNA
<213>  Artificial

<220>
<223>  attB2-nsP2SFV-Rev

<400>  14
ggggacaact ttgtacaaga aagttggtta acacccggcc gtgtgcat                  48

## Description

The present invention relates to a method to identify compounds, peptides or mutations that affect the virulence of *Alphaviruses,* in particular Chikungunya virus and related viruses from the Semliki Forest antigenic group and Sindbis virus. Specifically the current method uses differential expression of a reporter gene affected by a transcriptional shutoff mechanism mediated by the expression of a non-structural protein 2 (nsP2) *Alphavirus* protein, to identify compounds, peptides or mutations which have modulating effects upon the effects of expression of this.

*Alphaviruses* are a group of pathogens responsible for major public health problems. Members of this genus belong to the *Togaviridae* family and are transmitted by insects, in particular mosquitoes, to higher vertebrates including humans that serve as amplifying hosts (3). In insect vectors, they cause persistent lifelong infections without critically affecting the viability of the hosts. In contrast, vertebrate hosts usually develop an acute infection that often results in several disease symptoms prior to virus clearance by the immune system.

High levels of sequence similarity in the structural and nonstructural genes of all *Alphaviruses* studied so far strongly suggest that *Alphaviruses* have a common ancestor (11). However, *Alphaviruses* from the Old World and the New World have circulated independently for several centuries, and can be distinguished on the basis of their genomic sequences.

Eastern equine encephalitis (EEE) and Venezuelan equine encephalitis (VEE) viruses are two *Alphaviruses* from the New World lineage that cause fatal encephalitis in horses. This phylogenic group is also responsible for small outbreaks of encephalitis in humans and therefore represents a threat for human populations of Latin and North America.

Chikungunya virus, was responsible for the recent epidemic in Réunion Island (12), other Old World *Alphaviruses* from the "Semliki Forest" antigenic complex are responsible for painful arthralgia, polyarthritis, fever and sometimes rash in infected humans. In addition to Chikungunya virus and Semliki Forest virus, the Semliki Forest antigenic complex includes O'Nyong-nyong virus, Ross River virus and Mayaro virus (12). Sindbis virus is also associated with arthralgia, rash and fever, and originates from the Old World, but belongs to a separate antigenic complex.

Herein, the term Old World refers to those parts of the Earth known to Europeans, Asians and Africans in the 15th century before the voyages of Christopher Columbus; it includes Europe, Asia, and Africa (collectively known as Africa-Eurasia), plus surrounding islands. The term is in distinction from the New World, meaning the Americas and Australasia.

The genome of *Alphaviruses* is an 11.5-kb, single-stranded RNA of positive polarity that contains a 5' methylguanylate cap and a 3' polyadenylate tail (3). The genome is directly translated into the viral nonstructural proteins nsP1 to nsP4, which are encoded by the 5' two-thirds of the genome. These proteins form an enzymatic complex that ensures both the replication of the viral genome and the transcription of a subgenomic mRNA encoding for the structural proteins of the virus. This subgenomic mRNA is synthesized from a promoter located on the RNA anti-genome synthesized during replication. Together with genomic RNA, structural proteins assemble to form the viral particles.

The structural proteins are dispensable for RNA replication, and several *Alphavirus* genomes lacking structural genes replicate efficiently. This is because replication only relies on the 4 nonstructural proteins nsP1 to nsP4. The nonstructural proteins are synthesized as two polyproteins, termed nsP123 and nsP1234. nsP123 is produced when translation terminates at a STOP codon between nsP3 and nsP4, while reading through the STOP codon results in nsP1234. Both precursors are progressively processed into nsP1, nsP2, nsP3 and nsP4 by the protease domain of nsP2.

Negative-sense anti-genome RNA is synthesized early in infection by the nsP123 intermediate along with nsP4 or the nsP23 intermediate along with nsP1 and nsP4. Upon further processing by the protease domain of nsP2, the four nonstructural proteins form an enzymatic complex that synthesizes using the RNA anti-genome as a template, both the subgenomic mRNA encoding the structural proteins and the full-length positive-strand RNA genome. nsP2 is an essential component of the replication complex. As previously mentioned this protein contains a protease domain required for the sequential processing of the nonstructural polyprotein. RNA helicase and nucleotide triphosphatase activities have also been reported, and are directly involved in viral RNA replication (1).

Recently nsP2 from Sindbis virus was also shown to inhibit translation of cellular proteins, and transcription of both cellular mRNA and ribosomal RNAs (4, 5, 7).

Inhibition of the antiviral immune response by Semliki Forest virus and Ross River virus has also been investigated. In Semliki Forest virus-infected cells, a significant fraction of the nsP2 protein is transported to the nucleus. A mutant strain of Semliki Forest virus with an nsP2 protein defective for its nuclear translocation (SFV4-RDR) is attenuated for growth and deeply impaired for its ability to block IFN-β and TNF-α mRNA synthesis (2).

However, inhibition of cellular transcription and translation were equivalent between SFV4-RDR and the parental wild-type strain. Thus, inhibition of virus stress-inducible genes like IFN-β and TNF-α requires a fully functional nsP2 protein, whereas the general transcriptional shutoff tolerates mutations in the nuclear localization signal of nsP2. The transcriptional inhibition of several genes from the antiviral cluster was also reported when RAW264.7 macrophages were infected with Ross River virus (10).

Interestingly, the nuclear activity of transcription factors not associated with the antiviral response like human Sp-1 was not affected by Ross River virus infection. These observations suggest that Ross River virus inhibits cellular transcription in host cells, but this transcriptional shutoff is somehow specific to the antiviral, stress-inducible genes. It has not been demonstrated yet that nsP2 from Ross River is responsible for this transcriptional shutoff.

Moreover, sub-neutralizing concentrations of anti-Ross River virus antibodies were required to enhance the infection efficiency in these experiments, suggesting that IL-10 expression could also be induced by such immune-complexes, and inhibit antiviral gene expression.

Old World and New World *Alphaviruses* use different viral proteins to mediate transcriptional shutoff (6) of the host cell. EEE and VEE, two New World *Alphaviruses,* rely on their capsid to inhibit transcription in infected host cells, while Semliki Forest and Sindbis virus use nsP2. Altogether, these observations suggest that nsP2-mediated transcriptional shutoff maybe a general property of Old World *Alphaviruses.*

Given the great medical, financial and social effects of *Alphaviruses,* factors which modulate the virulence of these infective agents are of great interest. The inventors have developed a new methodology to identify both external agents such as compounds and peptides which affect aspects of *Alphavirus* virulence and the inventors new method also allows the identification of internal genetic modifications to the *Alphavirus* which modulate its virulence.

The invention concerns a method to identify modulators of virulence of an *Alphavirus,* comprising the steps:
(i) expressing an *Alphavirus* non-structural protein 2 (nsP2) coding sequence in at least one subject mammalian cell *in vitro;*
(ii-a) introducing a first reporter gene under the transcriptional control of an inducible promoter into the at least one subject mammalian cell *in vitro;*
(iii) monitoring the activity of this first reporter gene *in vitro;*
(iv) comparing said activity of the first reporter gene of step (iii) to at least one control mammalian cell not expressing the nsP2 coding sequence and comprising the first reporter gene under the transcriptional control of an inducible promoter *in vitro.*

The inventors therefore provide a new method which uses the inherent properties of the *Alphavirus* nsP2 gene product, which have been shown to have a direct effect upon *Alphavirus* virulence; in combination with a reporter gene to measure the effects of various factors upon the transcriptional repression of this reporter gene in comparison to the same reporter gene in the absence of nsP2 expression.

The inventors have used a mammalian cell line system to identify modulators of the effects of nsP2 mediated transcriptional repression as such modulators of this *in vitro* mammalian system would be likely to have a comparable effect *in vivo* and therefore be potentially useful as medicaments or tools in the further study of these important disease causing viruses.

Compounds or peptides which modulate the activity of the nsP2 protein will likely restore the transcription of the antiviral genes required for the clearing of the infection by the host.

In the current application the term modulate is taken to comprise any alteration to the measured effect of nsP2 expression upon one or more reporter genes. This modulation may for instance be to decrease or increase the effects of nsP2 expression.

In addition, *Alphaviruses* that carry mutations in the nsP2 protein and thereby have lost their ability to block cell transcription, will be attenuated and represent potential vaccine strains.

Preferably, the Old World *Alphavirus* for which virulence modulators are being identified is selected from the group consisting of Chikungunya virus, Semliki Forest virus, O'nyong-nyong virus, Sindbis virus, Mayaro virus, Ross River virus.

The inventors have shown for the first time in this application, that nsP2 from Chikungunya virus has a similar transcriptional repressive effect to nsP2 from Sindbis virus and Semliki Forest virus; their new observations suggest that nsP2-mediated transcriptional shutoff is a general property of *Alphaviruses* and therefore that their new method will be applicable to all *Alphaviruses.*

Preferably the nsP2 coding sequence is selected from the group comprising Sindbis virus nsP2 (SEQ ID NO: 4), Semliki Forest virus nsP2 (SEQ ID NO: 3) and Chikungunya virus nsP2 (SEQ ID NO: 1 or 2).

Preferably the first reporter gene produces a luminescent signal.

By using a reporter gene which produces a luminescent signal the effects of the various components of the current method can be observed and monitored at fixed time points and over time periods with a single population of cells.

More preferably the first reporter gene is a luciferase.

Luciferase is a generic name for enzymes commonly used in nature for bioluminescence. Several dozen commercially available luciferase enzymes exist with many more variants of these being available to meet the specific needs of a particular protocol.

Most preferably the luciferase is derived from *Photinus pyralis* luciferase.

The North American firefly *Photinus pyralis* has been used extensively as a reporter in studies of gene expression and therefore is considered by the inventors to be the best available reporter gene for use in the current method.

Preferably the method identifies modulators decreasing the virulence of an *Alphavirus.*

Modulators which decrease the virulence of *Alphaviruses* have potential use as medicaments in the treatment or alleviation of *Alphavirus* infection. In addition mutations to the *Alphavirus* which decrease its virulence would also make such modified *Alphavirus* particles potentially useful as attenuated viral vaccine candidates, and/or as model systems for their further study.

Modulators which decrease the virulence of the *Alphavirus* would be expected to increase the level of reporter gene activity relative to a control.

In the current application, a control or control experiment means any data set derived from an identical set of conditions as an experiment according to the method of the present application but lacking one or more components of experiment so allowing the effects of these components to be determined.

Alternatively the method identifies modulators increasing the virulence of an *Alphavirus.*

Modulators which increase the virulence of the *Alphavirus* are of potential interest as these may for instance give insights into one or more aspects of the virulence causing effects of the *Alphavirus* so allowing further study into new ways to treat such *Alphavirus* infections.

Preferably the method further comprises the expression of a second reporter gene under the control of a promoter unaffected by expression of said nsP2 coding sequence in said at least one subject mammalian cell *in vitro.*

The inventors provide a way of normalizing the efficiency of transfection of the various components of the current invention. By doing so it is possible to more accurately ascertain the effects of variable transfection rates and so more accurately interpret the activity of the first reporter gene and the effects of nsP2 expression thereon and the modulation which may be occurring.

By using a promoter which is not affected by expression of the nsP2 coding sequence, the effect of nsP2 expression can be removed and the transfection rate calculated more accurately as a result.

Preferably the promoter unaffected by expression of said nsP2 coding sequence is a viral promoter.

The inventors have found that viral promoters or elements thereof, appear to be less affected by expression of the nsP2 coding sequence than other promoter elements and therefore such viral promoters are suitable for use in expressing of a reporter gene of the transfection control.

In particular the viral promoter may be derived from the promoter of the Herpes Simplex Virus thymidine kinase gene.

The inventors have experimentally shown in the current application that the Herpes Simplex Virus thymidine kinase gene promoter was not significantly affected by nsP2 expression and therefore is suitable for use in the transfection control.

In the current application derived from, means any nucleic acid or protein sequence is created from an original sequence and then modified so as to retain its original functionality but have residue changes and/or additions or deletions relative to the original sequence.

Preferably the first reporter gene and the second reporter gene encode different products.

By using different reporter genes the activity of each of these can more easily be ascertained and so used to increase the usefulness of the current invention.

Most preferably the second reporter gene is derived from the *Renilla reniformis* luciferase gene.

The soft coral *Renilla reniformis* luciferase enzyme is a monomeric soluble intracellular protein that is used increasingly as a marker of gene expression, its luminescent output differs from that of *Photinus pyralis* luciferase and so is suitable for use as a transfection control in the current invention.

The present invention provides another method to identify modulators of virulence of an *Alphavirus,* wherein the nsP2 coding sequence comprises at least one alteration, deletion or addition mutation in comparison to a reference sequence selected from the group: SEQ ID NO: 1, SEQ ID: 2, SEQ ID NO: 3 or SEQ ID NO: 4.

The inventors therefore also provide the means to investigate the effects upon the virulence of an *Alphavirus* of alterations in the nsP2 coding sequence by way of least one alteration, deletion or addition mutation in comparison to a reference sequence for nsP2, which in accordance with this aspect of the present invention is the Chikungunya virus (SEQ ID NO: 1 or SEQ ID: 2), Sindbis virus (SEQ ID NO: 4) or Semliki Forest virus (SEQ ID NO: 3) wild type nsP2 coding sequence.

In the present invention wild type means the most common phenotype or genotype in the natural population.

Preferably the method comprises the analysis of a library of nsP2 coding sequences all having at least one alteration, deletion or addition mutation in comparison to a reference sequence selected from the group: SEQ ID NO: 1, SEQ ID: 2, SEQ ID NO: 3 or SEQ ID NO: 4.

The inventors also provide a method to investigate the effects upon the virulence of an Old World *Alphavirus* by studying a library of nsP2 mutants.

Preferably the library of nsP2 coding sequences result from a rational mutation strategy.

The inventors also provide a method of screening a library of rationally mutagenised nsP2 mutants. Recent advances in enzyme/protein engineering have used a combination of the random methods of directed evolution with elements of rational enzyme/protein modification to successfully by-pass certain limitations of both directed evolution and rational design. Semi-rational approaches that target multiple, specific residues to mutate on the basis of prior structural or functional knowledge create 'smart' libraries that are more likely to yield positive results.

Preferably the library of nsP2 coding sequences comprise mutations outside the protease domain.

Preferably the library of nsP2 coding sequences comprise mutations within the amino-terminal of said nsP2 coding sequence.

Preferably the method comprises a further step of:
(v) comparing said activity of the first reporter gene of step (iii) to a further at least one control mammalian cell expressing a wild type nsP2 coding sequence and comprising the first reporter gene under the transcriptional control of the inducible promoter.

The inventors therefore provide a method in which the effects of a mutagenised nsP2 coding sequence(s) upon the activity of a reporter gene are compared to the effects of a wild type nsP2 gene.

According to an advantageous embodiment of the current method, the inducible promoter comprises at least one virus induced stress response activated promoter element.

In the current application a virus induced stress response activated promoter element, refers to any genomic element which is transcriptionally activated in response to viral infection of its host cell.

The inventors have found that the nsP2 gene product can have a differential effect upon the transcriptional repression of genes associated with combating a viral infection and non-viral related gene transcription. By providing a reporter gene construction under the transcriptional control of at least one virus induced stress response activated promoter element these differential effects can be examined separately and therefore in more detail, allowing the identification of compounds and/or mutations which affect one or other type of transcription differently.

Preferably the at least one virus induced stress response promoter element is activated by at least one antiviral protein.

A number of proteins are known to be directly involved in a mammalian cell/mammals reaction to viral infection, these antiviral proteins in turn are also known to have a direct transcriptional effect upon certain other genes. Therefore the inventors provide a reporter gene under the control of such an element which is responsive to such an antiviral protein so allowing the specific investigation of modulators of nsP2s effect upon these important pathways.

Preferably the at least one antiviral protein is selected from the group comprising Interferon (IFN) type proteins and Tumor Necrosis Factor (TNF) type proteins.

Interferons (IFNs) and Tumour Necrosis Factors (TNF) are natural proteins produced by the cells of the immune system of most vertebrates in response to challenges by foreign agents such as viruses, bacteria, parasites and tumor cells. Interferons belong to a large class of glycoproteins known as cytokines. Interferons assist the immune response by inhibiting viral replication within other cells of the body. Tumor necrosis factors (or the TNF-family) refers to a group of cytokine families which can cause apoptosis. Both these families of molecules play crucial roles in the fighting of viral infections in mammalian cells and therefore further information concerning indirect modulators of there activity are likely to be very useful as medicaments and/or as research tools.

Most preferably the at least one antiviral protein is IFN-β.

The role and effects of IFN- β in mammalian cells during viral infections are well known and as indicated above information concerning indirect modulators of there activity are likely to be very useful as medicaments and/or as research tools, meaning this is an ideal element of the current method.

Preferably the method comprises before step (iii), the further steps of:
(ii-b) exposing at least one antiviral protein to the at least one subject mammalian cell; and
(v) comparing the activity of said first reporter gene of step (iii) to a further at least one control mammalian cell not exposed to the at least antiviral protein and comprising the first reporter gene under the transcriptional control of said inducible promoter.

The inventors therefore provide a method in which the effects of expression of a nsP2 coding sequence(s) upon the activity of a reporter gene under the control of at least one virus induced stress response activated promoter element exposed to an antiviral protein are compared to the effects upon one where the antiviral protein is not present.

In another embodiment of the present invention the inducible promoter comprises at least one transcription factor activated promoter element.

The inventors have found that the nsP2 gene product can have a differential effect upon the transcriptional repression of genes associated with combating a viral infection and normal gene transcription mediated by a transcription factor. By providing a reporter gene construct under the transcriptional control of at least one transcription factor activated promoter element, these differential effects can be examined separately and therefore in more detail.

In the present application a transcription factor activated promoter element, means any genomic element which is transcriptionally activated by its interaction with a transcription factor.

Preferably the at least one transcription factor activated promoter element comprises at least one DNA sequence binding target motif of the GAL4 transcription factor.

The DNA binding domain and characteristics of the GAL4 protein from *Saccharomyces cerevisiae* are extremely well known and have been characterised to an extraordinary degree. Methods using this information are wide spread, in particular the 'Two Hybrid' assay. Therefore the DNA sequence binding target motifs of the GAL4 protein in combination with the GAL4 DNA binding domain allow the implementation of a method according to the current invention.

Preferably the method comprises before step (iii), the further steps of:
(ii-a) expressing at least one hybrid protein comprising the GAL4 DNA binding domain fused to the transcription activation domain of at least one transcription factor, under the control of a promoter in the at least one subject mammalian cell; and
(v) comparing the activity of the first reporter gene of step (iii) to a further at least one control mammalian cell not expressing the at least one hybrid protein and comprising the first reporter gene under the transcriptional control of the inducible promoter.

An important feature of most eukaryotic transcription factors, is that the activating and binding domains are modular and can function in close proximity to each other without direct binding. This means that even though the transcription factor is split into two fragments, it can still activate transcription when the two fragments are indirectly connected. The important consequence of this in respect to current invention is that the GAL4 DNA binding domain can be fused to the transcription activation domain of a general Mammalian transcription factor and that this will lead to a hybrid protein with the combined properties of affinity for the GAL4 DNA binding target motifs and also the transcriptional activation of any gene associated with the GAL4 DNA binding target motifs.

Preferably at least one transcription factor is selected from the group comprising Jun, Fos and SMAD3.

Jun, Fos and SMAD3 are well known general mammalian transcriptional factors, which have been shown to affect overall transcription levels in mammalian cells. Therefore by providing hybrid proteins comprising fusions of the transcriptional activation domains of these proteins with the GAL4 DNA binding domain, the activity of a reporter gene associated with GAL4 DNA binding target motifs can be monitored and alterations in this used to determine overall changes in the transcription levels of a mammalian cell.

The present invention also provides a method to identify modulators of virulence of an *Alphavirus* comprising the further steps further steps of:
(ii-a) exposing at least one candidate molecule to the at least one subject mammalian cell; and
(v) comparing the activity of the first reporter gene of step (iii) to a further at least one control mammalian cell not exposed to the at least one candidate molecule and comprising the first reporter gene under the transcriptional control of said inducible promoter.

The inventors therefore provide a method of screening candidate modulator molecules using their new method.

Preferably the method involves the analysis of a library of candidate molecules.

The inventors also provide a method of screening a library of candidate modulator molecules using their new method.

Preferably the at least candidate molecule is selected from the group comprising peptides, glyco-peptides, lipo-peptides, pharmaceutically active compounds, carbohydrates, combinations and derivatives thereof.

In the present invention pharmaceutically active compounds, are defined as any chemical substance which can have a measurable effect upon the human or mammalian body when in a healthy or diseased state.

The inventors consider it possible to use any combination of the cis- or trans- reporter gene based transcriptional assays described herein to screen for mutations, compounds and/or peptides which affect the activity of nsP2 and the virulence of Alphaviruses.

There is also provided a kit of reagents to perform any one of the methods defined herein, comprising:
a first nucleic acid construct configured to express the nsP2 coding sequence in at least one subject mammalian cell, wherein this first nucleic acid construct comprises an nsP2 coding sequence under the transcriptional control of a promoter sequence;
a second nucleic acid construct configured to express the first reporter gene in at least one subject mammalian cell and at least one control mammalian cell, wherein this second nucleic acid construct comprises the coding sequence of a first reporter gene under the transcriptional control of an inducible promoter sequence;
reagents to introduce the first nucleic acid construct into at least one subject mammalian cell;
reagents to introduce the second nucleic acid constructs into at least one subject mammalian cell and at least one control mammalian cell;
reagents to monitor the activity of the first reporter gene;
instructions to perform the method.

The inventors therefore provide a kit of materials with which to perform a method according to the current invention

Preferably the kit of reagents further comprises:
a nucleic acid construct configured to express a second reporter gene in at least one subject mammalian cell and at least one control mammalian cell, wherein the nucleic acid construct comprises the coding sequence of a second reporter gene under the transcriptional control of a promoter unaffected by expression of the nsP2 coding sequence.

Preferably the nsP2 coding sequence comprises at least one alteration, deletion or addition mutation in comparison to a reference sequence SEQ ID NO: 1; and the kit further comprises:
a nucleic acid construct configured to express a wild type nsP2 coding sequence in a further at least one control mammalian cell, wherein this nucleic acid construct comprises the coding sequence of a wild type nsP2 coding sequence under the transcriptional control of a promoter sequence.

Preferably the inducible promoter sequence comprises at least one virus induced stress response activated promoter element.

Preferably the kit further comprises:
at least one antiviral protein reagent which is configured to activate the at least one virus induced stress response promoter element.

Alternatively inducible promoter sequence comprises at least one transcription factor activated promoter element.

Preferably the at least one transcription factor activated promoter element comprises at least one DNA sequence binding target motif of the GAL4 transcription factor.

Preferably the kit further comprises:
a nucleic acid construct configured to express a hybrid protein coding sequence in the at least one subject mammalian cell, wherein this nucleic acid construct comprises the coding sequence of the GAL4 DNA binding domain fused to the transcription activation domain of at least one transcription factor, under the control of a promoter.

There is also provided a composition for the modulation of a immunological response comprising nsP2, wherein the nsP2 coding sequence is selected from the group comprising Sindbis virus nsP2 (SEQ ID No: 4), Semliki Forest virus nsP2 (SEQ ID NO: 3) and Chikungunya virus nsP2 (SEQ ID NO: 1 or SEQ ID NO: 2). The nsP2 being in an administrable form with one or more pharmaceutically acceptable carriers or excipients.

There is also provided the use of an effective amount of a composition comprising nsP2, wherein said nsP2 coding sequence is selected from the group comprising Sindbis virus nsP2 (SEQ ID No: 4), Semliki Forest virus nsP2 (SEQ ID NO: 3) and Chikungunya virus nsP2 (SEQ ID NO: 1 or SEQ ID NO: 2) and one or more pharmaceutically acceptable carriers or excipients, for the preparation of a medicament for modulating an immunological response in a subject in need thereof.

The inventors also provide a composition comprising nsP2 and/or the use of nsP2 in the preparation of such a composition for the modulation of an immunological response in an individual. Given the effects of nsP2 described in the current application, upon both anti-viral specific and more general transcription levels, such nsP2 based compositions provide a means to modulate the immunological response of an individual by exposing this individual to compositions comprising this substance

For a better understanding of the invention and to show how the same may be carried into effect, there will now be shown by way of example only, specific embodiments, methods and processes according to the present invention with reference to the accompanying drawings in which:

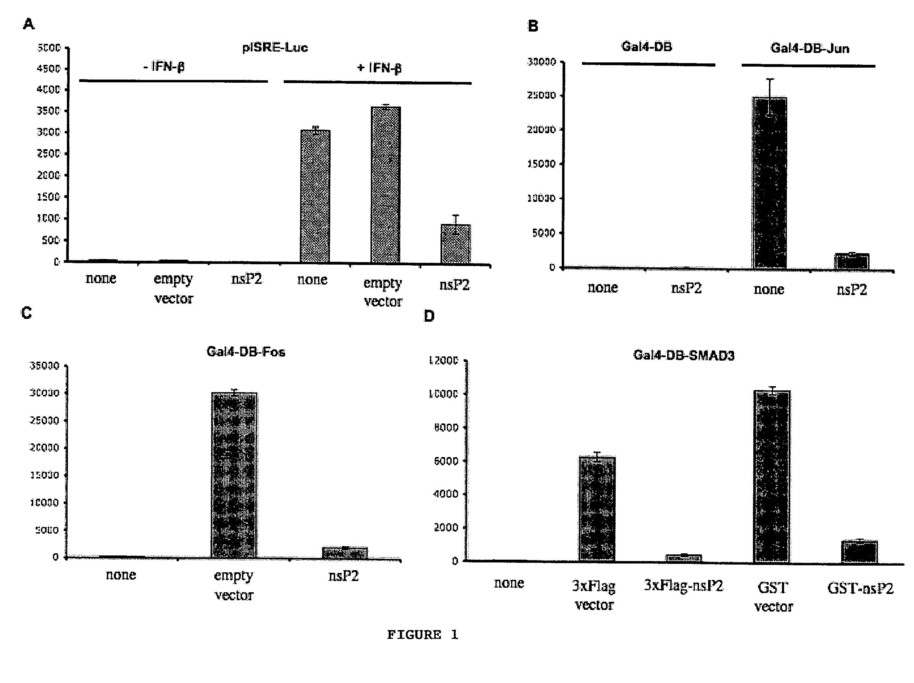

Figure 1: Panel A, shows the effects of nsP2 protein expression from Chikungunya virus upon the expression of a luciferase gene under the control of an Interferon-Stimulated Response Elements (ISRE) cis-regulatory element in the presence and absence of IFN-β.

Panel B shows the effects of nsP2 protein expression from Chikungunya virus upon the expression of a luciferase gene under the control of an GAL4-DB cis-regulatory element in the presence and absence of GAL4-Jun.

Panel C shows the effects of nsP2 protein expression from Chikungunya virus upon the expression of a luciferase gene under the control of an GAL4-DB cis-regulatory element in the presence and absence of GAL4-Fos.

Panel D shows the effects of nsP2 protein expression from Chikungunya virus upon the expression of a luciferase gene under the control of an GAL4-DB cis-regulatory element in the presence and absence of GAL4-SMAD3.

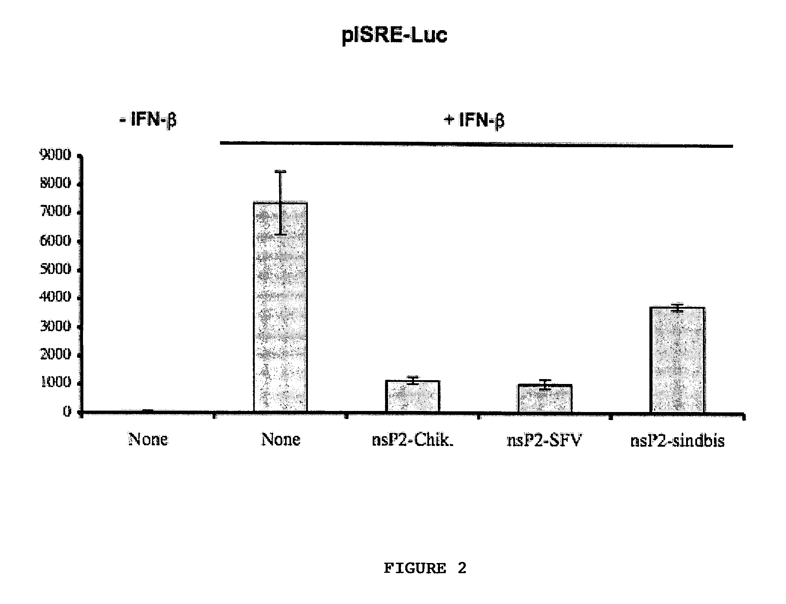

Figure 2: shows the effects of nsP2 protein expression from Chikungunya virus (nsP2-Chik), Semliki Forest virus (nsP2-SFV) and Sindbis virus (nsP2-sindbis) upon the expression of a luciferase gene under the control of an Interferon-Stimulated Response Elements (ISRE) cis-regulatory element in the presence and absence of IFN-β.

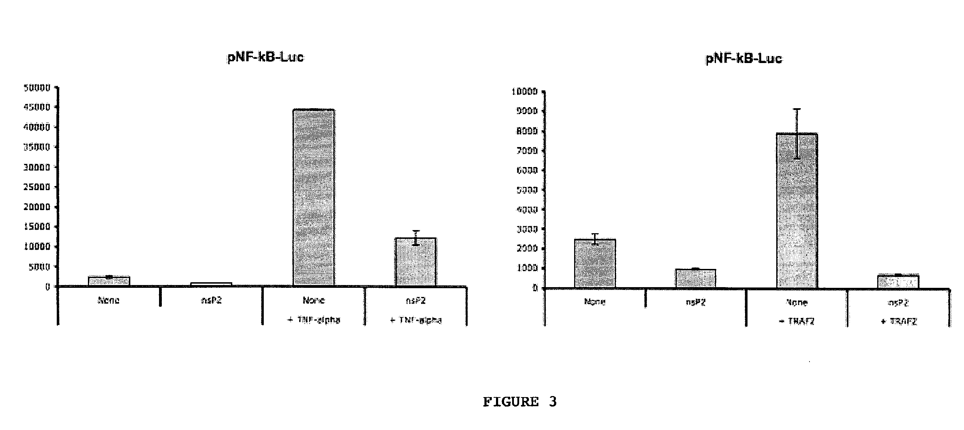

Figure 3: Panel A, shows the effects of nsP2 protein expression from Chikungunya virus upon the expression of a luciferase gene under the control of an the NFκB consensus sequence cis-regulatory element in the presence and absence of TNF-alpha (Tumour Necrosis Factor alpha).

Panel B shows the effects of nsP2 protein expression from Chikungunya virus upon the expression of a luciferase gene under the control of an NFκB consensus sequence cis-regulatory element in the presence and absence of TRAF2 (TNF receptor-associated factor 2).

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### EXAMPLE 1: Materials and Methods

According to a first specific example of the current invention, there is provided a screening method based on the expression, in mammalian cell lines, of a recombinant nsP2 protein (SEQ ID NO: 2) from Chikungunya virus. The expression of this protein inhibits the expression of a luciferase reporter gene driven by natural or synthetic hybrid transcription factors. Small molecules, peptides or nsP2 (SEQ ID NO: 2) mutations that restore the expression of the luciferase reporter gene represent different means to impair the transcriptional shutoff mediated by nsP2 from Chikungunya virus and related *Alphaviruses,* including members of the Semliki Forest antigenic complex and Sindbis virus.

The objective of the present screening method is to identify compounds, peptides or mutations that affect the virulence of *Alphaviruses* by targeting the transcriptional shutoff mediated by the nsP2 *Alphavirus* protein. To achieve this goal, the nsP2 protein from Chikungunya virus was cloned in a mammalian expression vector.

This DNA construct was co-transfected with a commercial reporter plasmid where the luciferase reporter gene is under control of Interferon-Stimulated Response Elements (ISRE). Although IFN-β efficiently induced luciferase expression, it was strongly inhibited by the addition of nsP2 protein from Chikungunya virus.

Mammalian cells were also transfected with a luciferase reporter construct containing, in its promoter, binding sites for the DNA-binding domain of GAL4 transcription factor (GAL4-DB). Hybrid transcription factors, composed of GAL4-DB fused to the transcription activation domains of Jun, Fos or SMAD3, induced luciferase expression. But co-expression with nsP2 from Chikungunya virus completely blocked this transcriptional activity. Such minimal *in vitro* systems are highly tractable, and suitable to achieve the screening of molecule libraries (protein-encoding cDNAs, chemical compounds) in a high-throughput setting as previously demonstrated (8, 9, 14-16). This system also provides a convenient method to screen nsP2 mutants, and identify those that fail to inhibit transcription.

### nsP2 expression plasmids

### From Chikungunya virus

The nsP2 encoding sequence from Chikungunya virus (isolate 05-115 from La Réunion Island) (13) was cloned by RT-PCR following manufacturer's recommendations (Titan One tube RT-PCR kit, ROCHE Boerhinger). The following nsP2-specific primers were used for the reverse transcription and amplification steps:
att-nsP2-For (SEQ ID NO: 4):
   5'-ggggacaactttgtacaaaaaagttggcatgGGAATAATAGAGACTCCGAGAGGAG-3'
attB2-nsP2-Rev (SEQ ID NO: 5):
   5'-ggggacaactttgtacaagaaagttggttaACATCCTGCTCGGGTGGC-3'

This PCR product was cloned with the Gateway^{®} recombination system (Invitrogen), primers were flanked by attB1 and attB2 Gateway^{®} recombination sequences (lower case on the primer sequences).

The RT-PCR product corresponding to nsP2 was introduced by *in vitro* recombination in pDONR™207 (Gateway^{®} system; Invitrogen) following manufacturer's recommendations. After selection of bacterial transformants on gentamicin-supplemented LB plate, isolated clones were picked and the nsP2 sequence was established by sequencing.

The sequence of the nsP2 clone used in this example almost matched the corresponding GenBank entry (AM258990, referred to as SEQ ID NO: 1 herein). The sequence of the nsP2 clone used in this example (SEQ ID NO:2) differed from SEQ ID NO: 1 by two nucleotide substitutions: one at position 26 (C to T; A9V mutation), and one at position 2378 (T to C; V739A) introduced by the attB2-nsP2-Rev primer (SEQ ID NO: 5).

The nsP2 encoding sequence was recombined into a mammalian expression vector compatible with the Gateway^{®} system, and expressed as a fusion protein with the GST or the 3xFLAG tag (Sigma-Aldrich) at their N-termini.

Mammalian expression was achieved using the pDEST27 vector for the GST fusion (Invitrogen), or a modified version of the pCI-neo plasmid (Promega) for the 3xFLAG tag. This later plasmid was built by introduction of the Gateway^{®} cassette (Gateway conversion system; Invitrogen) at the SmaI restriction site first, and subsequent introduction of the 3xFLAG-encoding sequence at the XhoI site.

### From Sindbis and Semliki Forest virus

The nsP2 encoding sequence from Sindbis virus (Genbank ID:
AAA96975) was cloned by PCR using pTOTO1101 plasmid (Rice C.M., Journal of Virology, Dec. 1987 p3809-3819) as a template and the following nsP2-specific primers:
   attB1-nsP2sinbis-For (SEQ ID NO 11):
      5'- ggggacaactttgtacaaaaaagttggcatgGCATTAGTTGAAACCCCG-3'
   attB2-nsP2sinbis-For (SEQ ID NO 12):
      5'- ggggacaactttgtacaagaaagttggttaGGCTCCAACTCCATCTCT-3'

The nsP2 encoding sequence from Semliki Forest virus (SFV) was cloned by PCR using plasmid (pSFV1, Invitrogen) as a template and the following nsP2-specific primers:
attB1-nsP2SFV-For (SEQ ID NO 13):
   5'- ggggacaactttgtacaaaaagttggcatgGGGGTCGTGGAAACACCT-3'
attB2-nsP2SFV-For (SEQ ID NO 14):
   5'- ggggacaactttgtacaagaaagttggttaACACCCGGCCGTGTGCAT-3'

To clone these PCR products with the Gateway^{®} recombination system (Invitrogen), primers were flanked by attB1 and attB2 Gateway^{®} recombination sequences (lower case on the primer sequences). The PCR products corresponding to nsP2 from Semliki Forest virus and Sindbis virus were introduced by *in vitro* recombination in pDONR™207 (Gateway^{®} system; Invitrogen) following manufacturer's recommendations. After selection of bacterial transformants on gentamicin-supplemented LB plate, isolated clones were picked and nsP2 sequence was established. The sequence of the nsP2 clones used in this study matched the corresponding GenBank entries.

### Cis- and Trans-reporter constructs

The pISRE-Luc Cis-Reporter plasmid was purchased from Stratagene. In this plasmid, the *Photinus pyralis* (firefly) luciferase gene is controlled by a synthetic promoter that contains direct repeats of the interferon-stimulated response element (ISRE). This response element is activated by stimulation of the cells with IFN-α or IFN-β.

The pNF-KB-luc CIS-reporter plasmid was purchased from Stratagene. pNFκB-Luc is designed to monitor the activation of NFκB signal transduction pathway. pNFκB-Luc contains the firefly luciferase (*luc*) gene from *Photinus pyralis* . This vector also contains multiple copies of the NFκB consensus sequence fused to a TATA-like promoter (*P*_{TAL}) region from the Herpes simplex virus thymidine kinase (HSV-TK) promoter. After endogenous NFκB proteins bind to the kappa (κ) enhancer element (κB), transcription is induced and the reporter gene is activated.

The luciferase coding sequence is followed by the SV40 late polyadenylation signal to ensure proper, efficient processing of the *luc* transcript in eukaryotic cells. Located upstream of NFκB is a synthetic transcription blocker (TB), which is composed of adjacent polyadenylation and transcription pause sites for reducing background transcription.

The DNA fragment encoding the transactivation domains of Jun (AA 1-223) SEQ ID NO: 7, Fos (AA 208-314) SEQ ID NO: 8 or SMAD3 (AA 156-425) SEQ ID NO: 9 were cloned by *in vitro* recombination in pDONR™207 (Invitrogen) using the Gateway^{®} system. These transactivation domains were subsequently introduced by recombination in a modified version of the pM vector (Clontech) made Gateway^{®} compatible inserting the Gateway^{®} cassette at the SmaI restriction site. Thereby, each transactivation domain could be expressed in mammalian cells as fusion proteins with the DNA binding domain of GAL4 (GAL4-DB) at their N-termini.

The DNA fragment encoding human TRAF2 (Genbank ID:
NP_066961) was cloned by *in vitro* recombination in pDONR™207 (Invitrogen) using the Gateway^{®} system. Like nsP2, this TRAF2 encoding sequence subsequently introduced by recombination in our modified Gateway-compatible 3x-FLAG vector to achieve expression in mammalian cells.

To measure the transactivation mediated by those constructs, the inventors used a reporter plasmid - pGAL4-UAS-Luc - containing the luciferase gene downstream of a promoter with Gal-DB binding sites (GAL4-UAS). The pGAL4-UAS-Luc vector is a modified version of the pGL3 vector (Promega) obtained by insertion at the SmaI site of a minimal promoter including five copies of the GAL4-UAS (underlined) and a TATA box (italicized). Nucleotide sequence of pGAL4-UAS-Luc minimal promoter:

GGGGAT-(CCGCTCGGAGGACAGTACT)5-CCGATCCGTCGACTCTAGAGG
GTATATAATGCGCCAGCTCGAATT (SEQ ID NO: 10)

The pRL-TK vector (Promega) was used as a reference reporter plasmid to normalize the efficiency of the transfection in all the experiments described below. This vector contains the herpes simplex virus thymidine kinase (HSV-TK) promoter just upstream of the *Renilla* luciferase gene. Inhibition of this reporter construct by the nsP2 protein of Chikungunya virus was tested, and was found to be non-significant.

### Cells

Human embryonic kidney cells 293T (HEK-293T; ATCC) were maintained in Dulbecco's modified Eagle's medium (DMEM; Gibco-Invitrogen) containing 10% fetal bovine serum, penicillin, and streptomycin at 37 °C and 5% CO2. Transfection was performed with Lipofectamine 2000 (Invitrogen) according to manufacturer's recommendation.

### ISRE cis-reporter gene assay results

HEK-293T cells were plated in 24-well plates (2x10⁵ per well). One day later, cells were transfected with the pISRE-Luc reporter plasmid (0,3 µg/well), the pRL-TK reference plasmid (0.03 µg/well), and the 3xFLAG-nsP2 construct or the corresponding empty vector (0,3 µg/well). After 24 h, IFN-β (Biosource) was added to the medium at 1000 IU/ml, and the cells were incubated for 24 h. The cells were lysed, and the firefly luciferase and *Renilla* luciferase activities in the lysate were measured using the Dual-luciferase Reporter Assay System (Promega) following manufacturer's recommendation. Reporter activity was calculated as the ratio of firefly luciferase activity to reference *Renilla* luciferase activity. As shown in Fig. 1A, nsP2 from Chikungunya virus strongly inhibited the expression of the IFN-β-induced ISRE-luciferase reporter gene. As shown in Fig. 2, nsP2 from Semliki Forest and Sindbis virus inhibited the expression of the IFN-β-induced ISRE-luciferase reporter gene.

Thus, this system can be used to screen compound libraries, peptides or nsP2 mutants, and identify those that restore cellular response to type I interferons for a least three *alphaviruses*: Chikungunya virus, Semliki Forest virus and Sindbis virus.

Experiments were performed in triplicates, and data represent means ± SD. Reporter activity is expressed as the ratio of firefly to *Renilla* luciferase activities.

HEK-293T cells were plated in 24-well plates (2x10⁵ per well). One day later, cells were transfected with the pNF-kB-Luc reporter plasmid (0,3 µg/well), the pRL-TK reference plasmid (0.03 µg/well), and the 3xFLAG-nsP2 construct or the corresponding empty vector (0,3 µg/well). After 24 h, TNF-α (R&D Systems) was added to the medium at 10 ng/ml, and the cells were incubated for 24 h. The cells were lysed, and the firefly luciferase and *Renilla* luciferase activities in the lysate were measured using the Dual-luciferase Reporter Assay System (Promega) following manufacturer's recommendation. Reporter activity was calculated as the ratio of firefly luciferase activity to reference *Renilla* luciferase activity. As shown in Fig. 3A, nsP2 from Chikungunya virus strongly inhibited the expression of the TNF-α-induced NF-kB-luciferase reporter gene. Thus, this system can be used to screen compound libraries, peptides or nsP2 mutants, and identify those that restore cellular response to TNF-α. Because TRAF2 is a key component of the TNF-α signalling pathway that activates NF-kB transcription factor, the ability of nsP2 from Chikungunya virus to block TRAF2-induced NF-kB-luciferase reporter gene was tested. HEK-293T cells were plated in 24-well plates (2x10⁵ per well). One day later, cells were transfected with the pNF-kB-Luc reporter plasmid (0,3 µg/well), the pRL-TK reference plasmid (0.03 µg/well), the 3xFLAG-TRAF2 plasmid, and the 3xFLAG-nsP2 construct or the corresponding empty vector (0,3 µg/well). After 48 h, the firefly to *Renilla* luciferase activities were determined as described. As shown in Fig. 3B, nsP2 from Chikungunya virus strongly inhibited the expression of the TRAF2-induced NF-kB-luciferase reporter gene. Thus, nsP2 from Chikungunya virus inhibits some key step of the TNF-α signaling pathway downstream of the TRAF2 element.

### Jun, Fos and SMAD3 trans-reporter gene assays results

HEK-293T cell s were plated in 24-well plates (2x10⁵ per well). One day later, cells were transfected with the pGAL4-UAS-Luc reporter plasmid (0,3 µg/well), the pRL-TK reference plasmid (0.03 µg/well), specified nsP2-encoding plasmids or the corresponding empty vector (0,3 µg/well), and pM vectors encoding GAL4-DB fused to the transactivation domain of either Jun SEQ ID NO: 7, Fos SEQ ID NO: 8 or SMAD3 SEQ ID NO: 9. As shown in Fig 1B, GAL4-DB-Jun transactivated the expression of the luciferase reporter gene, and this activity was strongly inhibited by the nsP2 protein of Chikungunya virus. Similar results were obtained when GAL4-DB-Fos or GAL4-DB-SMAD3 were the transactivating factors (Fig. 1C and 1D). This system can be used to screen compound libraries, peptides or nsP2 mutants, and identify those that restore cellular response to subsequent to the activation of Jun, Fos or SMAD3.

Experiments were performed in triplicates, and data represent means ± SD. Reporter activity is expressed as the ratio of firefly to *Renilla* luciferase activities.

### REFERENCES

1. Balistreri, G., J. Caldentey, L. Kaariainen, and T. Ahola. 2007. Enzymatic defects of the nsP2 proteins of Semliki Forest virus temperature-sensitive mutants. J Virol 81:2849-60.
2. Breakwell, L., P. Dosenovic, G. B. Hedestam, M. D'Amato, P. Liljestrom, J. Fazakerley, and G. M. McInerney. 2007. Semliki Forest virus nonstructural protein 2 is involved in suppression of the type I interferon response. J Virol 81:8677-84.
3. Fields, B. N., D. M. Knipe, P. M. Howley, and D. E. Griffin. 2001. Fields' virology, 4th ed. Lippincott Williams & Wilkins, Philadelphia.
4. Frolova, E. I., R. Z. Fayzulin, S. H. Cook, D. E. Griffin, C. M. Rice, and I. Frolov. 2002. Roles of nonstructural protein nsP2 and Alpha/Beta interferons in determining the outcome of Sindbis virus infection. J Virol 76:11254-64.
5. Garmashova, N., R. Gorchakov, E. Frolova, and I. Frolov. 2006. Sindbis virus nonstructural protein nsP2 is cytotoxic and inhibits cellular transcription. J Virol 80:5686-96.
6. Garmashova, N., R. Gorchakov, E. Volkova, S. Paessler, E. Frolova, and I. Frolov. 2007. The Old World and New World Alphaviruses use different virus-specific proteins for induction of transcriptional shutoff. J Virol 81:2472-84.
7. Gorchakov, R., E. Frolova, and I. Frolov. 2005. Inhibition of transcription and translation in Sindbis virus-infected cells. J Virol 79:9397-409.
8. Gu, B., S. Ouzunov, L. Wang, P. Mason, N. Bourne, A. Cuconati, and T. M. Block. 2006. Discovery of small molecule inhibitors of West Nile virus using a high-throughput sub-genomic replicon screen. Antiviral Res 70:39-50.
9. Liu, Y., J. T. Kern, J. R. Walker, J. A. Johnson, P. G. Schultz, and H. Luesch. 2007. A genomic screen for activators of the antioxidant response element. Proc Natl Acad Sci U S A 104:5205-10.
10. Mahalingam, S., and B. A. Lidbury. 2002. Suppression of lipopolysaccharide-induced antiviral transcription factor (STAT-1 and NF-kappa B) complexes by antibody-dependent enhancement of macrophage infection by Ross River virus. Proc Natl Acad Sci U S A 99:13819-24.
11. Powers, A. M., A. C. Brault, Y. Shirako, E. G. Strauss, W. Kang, J. H. Strauss, and S. C. Weaver. 2001. Evolutionary relationships and systematics of the Alphaviruses. J Virol 75:10118-31.
12. Powers, A. M., and C. H. Logue. 2007. Changing patterns of chikungunya virus: re-emergence of a zoonotic arbovirus. J Gen Virol 88:2363-77.
13. Schuffenecker, I., I. Iteman, A. Michault, S. Murri, L. Frangeul, M. C. Vaney, R. Lavenir, N. Pardigon, J. M. Reynes, F. Pettinelli, L. Biscornet, L. Diancourt, S. Michel, S. Duquerroy, G. Guigon, M. P. Frenkiel, A. C. Brehin, N. Cubito, P. Despres, F. Kunst, F. A. Rey, H. Zeller, and S. Brisse. 2006. Genome microevolution of chikungunya viruses causing the Indian Ocean outbreak. PLoS Med 3:e263.
14. Sohn, T. A., R. Bansal, G. H. Su, K. M. Murphy, and S. E. Kern. 2002. High-throughput measurement of the Tp53 response to anticancer drugs and random compounds using a stably integrated Tp53-responsive luciferase reporter. Carcinogenesis 23:949-57.
15. Sohn, T. A., G. H. Su, B. Ryu, C. J. Yeo, and S. E. Kern. 2001. High-throughput drug screening of the DPC4 tumor-suppressor pathway in human pancreatic cancer cells. Ann Surg 233:696-703.
16. Su, G. H., T. A. Sohn, B. Ryu, and S. E. Kern. 2000. A novel histone deacetylase inhibitor identified by high-throughput transcriptional screening of a compound library. Cancer Res 60:3137-42.

## Claims

1. A method to identify modulators of virulence of an *Alphavirus,* comprising the steps:
(i) expressing an *Alphavirus* non-structural protein 2 (nsP2) coding sequence in at least one subject mammalian cell *in vitro;*
(ii-a) introducing a first reporter gene under the transcriptional control of an inducible promoter into said at least one subject mammalian cell *in vitro;*
(iii) monitoring the activity of said first reporter gene *in vitro*;
(iv) comparing said activity of said first reporter gene of step (iii) to at least one control mammalian cell not expressing said nsP2 coding sequence and comprising said first reporter gene under the transcriptional control of said inducible promoter *in vitro.*

2. The method to identify modulators of virulence of an *Alphavirus* as claimed in claim 1, wherein said *Alphavirus* is selected from the group consisting of Chikungunya virus, Semliki Forest virus, O'nyong-nyong virus, Sindbis virus, Mayaro virus, Ross River virus.

3. The method to identify modulators of virulence of an *Alphavirus* as claimed in claim 1 or 2, wherein said nsP2 coding sequence is selected from the group comprising Sindbis virus nsP2 (SEQ ID No: 4), Semliki Forest virus nsP2 (SEQ ID NO: 3) and Chikungunya virus nsP2 (SEQ ID NO: 1 or SEQ ID NO: 2).

4. The method to identify modulators of virulence of an *Alphavirus* as claimed in any one of claims 1, 2 or 3, wherein said first reporter gene produces a luminescent signal.

5. The method to identify modulators of virulence of an *Alphavirus* as claimed in claim 4, wherein said first reporter gene is a luciferase.

6. The method to identify modulators of virulence of an *Alphavirus* as claimed in claim 5, wherein said luciferase is derived from *Photinus pyralis* luciferase.

7. The method to identify modulators of virulence of an *Alphavirus* as claimed in any one of claims 1 to 6, wherein said method identifies modulators decreasing the virulence of an *Alphavirus.*

8. The method to identify modulators of virulence of an *Alphavirus* as claimed in any one of claims 1 to 6, wherein said method identifies modulators increasing the virulence of an *Alphavirus.*

9. The method to identify modulators of virulence of an *Alphavirus* as claimed in any one of claims 1 to 8, wherein said method further comprises the expression of a second reporter gene under the control of a promoter unaffected by expression of said nsP2 coding sequence in said at least one subject mammalian cell *in vitro.*

10. The method to identify modulators of virulence of an *Alphavirus* as claimed in claim 9, wherein said promoter unaffected by expression of said nsP2 coding sequence is a viral promoter.

11. The method to identify modulators of virulence of an *Alphavirus* as claimed in claim 10, wherein said viral promoter is derived from the promoter of the Herpes Simplex Virus thymidine kinase gene.

12. The method to identify modulators of virulence of an *Alphavirus* as claimed in claim 9, 10 or 11, wherein said first reporter gene and said second reporter gene encode different products.

13. The method to identify modulators of virulence of an *Alphavirus* as claimed in any one of claims 9 to 12, wherein said second reporter gene is derived from the *Renilla reniformis* luciferase gene.

14. The method to identify modulators of virulence of an *Alphavirus* as claimed in any one of claims 1 to 13, wherein said nsP2 coding sequence comprises at least one alteration, deletion or addition mutation in comparison to a reference sequence selected from the group: SEQ ID NO: 1, SEQ ID: 2, SEQ ID NO: 3 or SEQ ID NO: 4.

15. The method to identify modulators of virulence of an *Alphavirus* as claimed in claim 14, wherein said method comprises the analysis of a library of nsP2 coding sequences all having at least one alteration, deletion or addition mutation in comparison to a reference sequence selected from the group: SEQ ID NO: 1, SEQ ID: 2, SEQ ID NO: 3 or SEQ ID NO: 4.

16. The method to identify modulators of virulence of an *Alphavirus* as claimed in claim 15, wherein said library of nsP2 coding sequences result from a rational mutation strategy.

17. The method to identify modulators of virulence of an *Alphavirus* as claimed in claim 16, wherein said library of nsP2 coding sequences comprise mutations outside the protease domain.

18. The method to identify modulators of virulence of an *Alphavirus* as claimed in claim 16 or 17, wherein said library of nsP2 coding sequences comprise mutations within the amino-terminal of said nsP2 coding sequence.

19. The method to identify modulators of virulence of an *Alphavirus* as claimed in any one of claims 14 to 18, wherein said method comprises a further step of:
(v) comparing said activity of said first reporter gene of step (iii) to a further at least one control mammalian cell expressing a wild type nsP2 coding sequence and comprising said first reporter gene under the transcriptional control of said inducible promoter.

20. The method to identify modulators of virulence of an *Alphavirus* as claimed in any one of claims 1 to 19, wherein said inducible promoter comprises at least one virus induced stress response activated promoter element.

21. The method to identify modulators of virulence of an *Alphavirus* as claimed in claim 20, wherein said at least one virus induced stress response promoter element is activated by at least one antiviral protein.

22. The method to identify modulators of virulence of an *Alphavirus* as claimed in claim 21, wherein said at least one antiviral protein is selected from the group comprising Interferon (IFN) type proteins and Tumour Necrosis Factor (TNF) type proteins.

23. The method to identify modulators of virulence of an *Alphavirus* as claimed in claim 22, wherein said at least one antiviral protein is selected from the group consisting of IFN-β, TNF and TRAF2.

24. The method to identify modulators of virulence of an *Alphavirus* as claimed in any one of claims 20 to 23, wherein said method comprises before step (iii), the further steps of:
(ii-b) exposing at least one antiviral protein to said at least one subject mammalian cell; and
(v) comparing said activity of said first reporter gene of step (iii) to a further at least one control mammalian cell not exposed to said at least antiviral protein and comprising said first reporter gene under the transcriptional control of said inducible promoter.

25. The method to identify modulators of virulence of an *Alphavirus* as claimed in any one of claims 1 to 19, wherein said inducible promoter comprises at least one transcription factor activated promoter element.

26. The method to identify modulators of virulence of an *Alphavirus* as claimed in claim 25, wherein said at least one transcription factor activated promoter element comprises at least one DNA sequence binding target motif of the GAL4 transcription factor.

27. The method to identify modulators of virulence of an *Alphavirus* as claimed in claim 25 or 26, wherein said method comprises before step (iii), the further steps of:
(ii-b) expressing at least one hybrid protein comprising the GAL4 DNA binding domain fused to the transcription activation domain of at least one transcription factor, under the control of a promoter in said at least one subject mammalian cell; and
(v) comparing said activity of said first reporter gene of step (iii) to a further at least one control mammalian cell not expressing said at least one hybrid protein and comprising said first reporter gene under the transcriptional control of said inducible promoter.

28. The method to identify modulators of virulence of an *Alphavirus* as claimed in claim 26 or 27, wherein said at least one transcription factor is selected from the group comprising Jun, Fos and SMAD3.

29. The method to identify modulators of virulence of an *Alphavirus* as claimed in any one of claims 1 to 28, wherein said method comprises before step (iii), the further steps of:
exposing at least one candidate molecule to said at least one subject mammalian cell; and
comparing said activity of said first reporter gene of step (iii) to a further at least one control mammalian cell not exposed to said at least one candidate molecule and comprising said first reporter gene under the transcriptional control of said inducible promoter.

30. The method to identify modulators of virulence of an *Alphavirus* as claimed in claim 29, wherein said method involves the analysis of a library of candidate molecules.

31. The method to identify modulators of virulence of an *Alphavirus* as claimed in claim 29 or 30, wherein said at least candidate molecule is selected from the group comprising peptides, glyco-peptides, lipo-peptides, pharmaceutically active compounds, carbohydrates, combinations and derivatives thereof.

32. A kit of reagents to perform a method according to any one of claims 1 to 31, comprising:
a first nucleic acid construct configured to express said nsP2 coding sequence in said at least one subject mammalian cell, wherein said first nucleic acid construct comprises said nsP2 coding sequence under the transcriptional control of a promoter sequence;
a second nucleic acid construct configured to express said first reporter gene in said at least one subject mammalian cell and said at least one control mammalian cell, wherein said second nucleic acid construct comprises the coding sequence of said first reporter gene under the transcriptional control of an inducible promoter sequence;
reagents to introduce said first nucleic acid constructs into said at least one subject mammalian cell;
reagents to introduce said second nucleic acid constructs into said at least one subject mammalian cell and said at least one control mammalian cell;
reagents to monitor the activity of said first reporter gene;
instructions to perform the method.

33. A kit of reagents as claimed in claim 32, further comprising:
a nucleic acid construct configured to express said second reporter gene in said at least one subject mammalian cell and said at least one control mammalian cell, wherein said nucleic acid construct comprises the coding sequence of said second reporter gene under the transcriptional control of a promoter unaffected by expression of said nsP2 coding sequence.

34. A kit of reagents as claimed in claim 32 or 33, wherein said nsP2 coding sequence comprises wherein comprises at least one alteration, deletion or addition mutation in comparison to a reference sequence SEQ ID NO: 1, SEQ ID NO: 2, SEQ ID NO: 3, SEQ ID NO: 4; and said kit further comprises:
a nucleic acid construct configured to express a wild type nsP2 coding sequence in a further at least one control mammalian cell, wherein said nucleic acid construct comprises the coding sequence of said wild type nsP2 coding sequence under the transcriptional control of a promoter sequence.

35. A kit of reagents as claimed in any one of claims 32, 33 or 34, wherein said inducible promoter sequence comprises at least one virus induced stress response activated promoter element.

36. A kit of reagents as claimed in claim 35, wherein said kit further comprises:
at least one antiviral protein reagent which is configured to activate said at least one virus induced stress response promoter element.

37. A kit of reagents as claimed in any one of claims 32, 33 or 34, wherein said inducible promoter sequence comprises at least one transcription factor activated promoter element.

38. A kit of reagents as claimed in any one of claims 37, wherein said at least one transcription factor activated promoter element comprises at least one DNA sequence binding target motif of the GAL4 transcription factor.

39. A kit of reagents as claimed in claim 37 or 38, wherein said kit further comprises:
a nucleic acid construct configured to express a hybrid protein coding sequence in said at least one subject mammalian cell, wherein said nucleic acid construct comprises the coding sequence of the GAL4 DNA binding domain fused to the transcription activation domain of at least one transcription factor, under the control of a promoter.

40. A composition for the modulation of a immunological response comprising isolated, natural or synthetic nsP2 or an active fragment thereof, wherein said nsP2 coding sequence is selected from the group comprising Sindbis virus nsP2 (SEQ ID No: 4), Semliki Forest virus nsP2 (SEQ ID NO: 3) and Chikungunya virus nsP2 (SEQ ID NO: 1 or SEQ ID NO: 2) in an administrable form with one or more pharmaceutically acceptable carriers or excipients.

41. Use of an effective amount of a composition comprising isolated, natural or synthetic nsP2 or an active fragment thereof, wherein said nsP2 coding sequence is selected from the group comprising Sindbis virus nsP2 (SEQ ID No: 4), Semliki Forest virus nsP2 (SEQ ID NO: 3) and Chikungunya virus nsP2 (SEQ ID NO: 1 or SEQ ID NO: 2) and one or more pharmaceutically acceptable carriers or excipients, for the preparation of a medicament for modulating an immunological response in a subject in need thereof.
